(19) Europäisches Patentamt / European Patent Office / Office européen des brevets

(11) **EP 1 708 636 B1**

(12) **EUROPEAN PATENT SPECIFICATION**

(45) Date of publication and mention
of the grant of the patent:
**26.07.2017 Bulletin 2017/30**

(51) Int Cl.:
*A61B 18/14* *(2006.01)*    *A61B 18/18* *(2006.01)*

(21) Application number: **05705201.1**

(22) Date of filing: **07.01.2005**

(86) International application number:
**PCT/US2005/000432**

(87) International publication number:
**WO 2005/070316 (04.08.2005 Gazette 2005/31)**

(54) **SYSTEM FOR TREATING ABNORMAL EPITHELIUM IN AN ESOPHAGUS**

SYSTEM ZUR BEHANDLUNG VERÄNDERTEN EPITHELS IN EINEM ÖSOPHAGUS

SYSTÈME SERVANT À TRAITER UN ÉPITHÉLIUM ANORMAL DANS L'OESOPHAGE

(84) Designated Contracting States:
**AT BE BG CH CY CZ DE DK EE ES FI FR GB GR HU IE IS IT LI LT LU MC NL PL PT RO SE SI SK TR**

(30) Priority: **09.01.2004 US 754445**

(43) Date of publication of application:
**11.10.2006 Bulletin 2006/41**

(73) Proprietor: **Covidien LP
Mansfield, MA 02048 (US)**

(72) Inventors:
 • **GANZ, Robert A.
Minneapolis, MN 55416 (US)**
 • **ZELICKSON, Brian D.
Minneapolis, MN 55416 (US)**
 • **STERN, Roger A.
Cupertino, CA 95014 (US)**
 • **JACKSON, Jerome
Los Altos, CA 94024 (US)**
 • **SMITH, George H.
Palo Alto, CA 94301 (US)**

(74) Representative: **Maschio, Antonio et al
Maschio & Soames IP Limited
30 Carlton Crescent
Southampton SO15 2EW (GB)**

(56) References cited:
**WO-A1-01/22897         WO-A2-03/092609
US-A- 4 930 521         US-A- 6 023 638
US-A- 6 033 397         US-A- 6 112 123
US-A1- 2002 156 470     US-B1- 6 405 732**

**Description**

BACKGROUND OF THE INVENTION

1. Field of the Invention. A system for treating abnormal epithelium in an esophagus.

Two of the major functions of the human esophagus are the transport of food from intake to the stomach and the prevention of retrograde flow of gastrointestinal contents. The retrograde flow is, in part, prevented by two esophageal sphincters which normally remain closed and which are functional rather than distinct entities. In particular, a lower esophageal sphincter normally remains closed until parasympathetic activation causes its relaxation, allowing food to pass into the stomach from the esophagus. Various types of food and other activity may cause relaxation of the sphincter, such as fatty meals, smoking and beverages having xanthene content. Certain drugs or pharmaceuticals also may cause relaxation of this lower esophageal sphincter, as well as localized trauma or other problems such as neuromuscular disorders.

Regardless, patients having such difficulties may present with clinical indications including dysphagia, or difficulty in swallowing, as well as more classic symptoms of heartburn and other similar complaints. Recurrent problems of this nature often lead to a disorder known as reflux esophagitis, consisting of esophageal mucosa damage due to the interaction of the gastric or intestinal contents with portions of the esophagus having tissue not designed to experience such interaction. As suggested above, the causative agent for such problems may vary.

WO03/092609(A2) describes a method, and system, for treating a tissue site of a tissue structure that has at least a first and a second tissue plane. An energy delivery device is provided. A barrier is created between the first and second tissue planes. At least a portion of an energy delivery device is positioned at the tissue site. Sufficient energy is delivered from the energy delivery device to create cell necrosis of at least a portion of the first tissue plane.

US 6 405 732 B1 relates to a method to treat gastric reflux via the detection and ablation of gastro-esophageal nerves and receptors.

BRIEF SUMMARY OF THE INVENTION

Particular aspects of the invention are defined in the claims.

A treatment catheter containing an energy delivery device and method of use are provided to access and treat portions of the human esophagus, particularly those suffering from or at risk of experiencing undesired growth of columnar epithelium. The catheter system and method provides for a controlled depth of injury and/or ablation of the mucosal layer of the esophagus, typically using radiofrequency energy, non-ionizing ultraviolet radiation, microwave radiation, thermal energy (including both heating and cooling), ultrasonic energy, light energy (optionally combined with the use of photosensitizers), and the like. The present invention is particularly intended and adapted to treat the mucosal layer of the esophageal wall with minimal or no damage to the underlying submucosal layer. In this way, a normal mucosal layer, i.e., substantially free from metaplastic and other damaged cells characteristic of disorders, such as Barrett's esophagus or metaplasia can regenerate. In particular, by ablating the mucosal layer of the esophagus and suppressing or eliminating acid intrusion into the lower esophagus over the esophageal sphincter during healing, the injured or necrosed mucosal cells will be replaced with normal mucosal cellular growth.

Thus, in a comparative example, a method for treating abnormal mucosa in an esophagus comprises positioning an energy delivery device within the esophagus. Energy is delivered from the device under conditions selected to injure or necrose the mucosal layer and to initiate regrowth of a normal mucosal layer without causing substantial injury to the submucosal layer underlying the mucosal layer. Typically, the total energy per square cm delivered to the esophageal tissue will be in the range from 1 J/cm$^2$ to 50 J/cm$^2$, usually being from 5 J/cm$^2$ to 15 J/cm$^2$. In order to effectively ablate the mucosal lining of the esophagus and allow re-growth of a normal mucosal lining without creating damage to underlying tissue structures, it is preferable to deliver the radiofrequency energy over a short time span in order to reduce the effects of thermal conduction of energy to deeper tissue layers, thereby creating a "searing" effect. It is preferable to deliver the radiofrequency energy within a time span of less than 5 seconds. An optimal time for effective treatment is less than 1 second, and preferably less than 0.5 second or 0.25 seconds. The lower bound on time may be limited by the ability of the RF-power source to deliver high powers. Since the electrode area and consequently the tissue treatment area can be as much as several square centimeters, RF powers of several hundred watts would be required in order to deliver the desired energy density in short periods of time. This may pose a practical limitation on the lower limit of time. However, an RF power source configured to deliver a very short, high power, pulse of energy could be utilized. Using techniques similar to those used for flash lamp sources, or other types of capacitor discharge sources, a very high power, short pulse of RF energy can be created. This would allow treatment times of a few milliseconds or less, e.g. less than about 5 milliseconds. While this type of approach is feasible, in practice a more conventional RF source with a power capability of several hundred watts may be preferred.

Thus, for a power supply with a given maximum power capability, the time (t) needed to deliver the targeted total energy dosage (E) may be calculated by dividing the total energy by the available power (P), where the total energy dosage E is equal to the targeted energy density per unit area multiplied by the treatment area.

$$t = \frac{E}{P}$$

It is presently believed that energy delivery under these conditions will raise the temperature of the mucosal tissue to a temperature sufficient to disable the cell replication mechanisms, typically from about 65°C to 85°C, without raising the temperature of the submucosal tissues to that level. Such selective heating is achieved by rapid delivery of relatively high power levels. While this heating profile is offered to help explain the underlying mechanism of the present disclosure the mechanism may not be entirely accurate and is not essential to the performance or success of the present disclosure.

In order to treat Barrett's esophagus or metaplasia, the energy delivery device will usually be positioned within the lower esophagus at a location just above the lower esophageal sphincter (LES). Usually, the energy delivery device will be positioned to deliver energy around a circumferential mucosal surface having a length in the axial direction, within the esophagus of from 1 cm to 15 cm, usually from 1 cm to 8 cm, located above the lower esophageal sphincter. The length of the area of the esophagus to treat above the LES will usually be determined by visual examination using an endoscope. The treatment will be effected using an energy applicator in one or more treatment steps, depending on the length of the applicator head which delivers energy.

The energy may be delivered circumferentially about the axis of the energy-delivering device in a single step, i.e., all at one time. Alternatively, the energy may be delivered to different circumferential and/or axial sections of the esophageal wall sequentially. In either case, the energy may be delivered in an amount and over a time sufficient to necrose tissue or, alternatively, sufficient only to injure the mucosal layer without substantial necrosis. Preferably, injury to the underlying submucosal region will be limited or avoided entirely. In particular, it is desirable to limit the depth of injury or necrosis to the submucosal and deeper tissues to permit rapid healing and to avoid stricture formation in theses tissues.

In a first example the energy delivered to the esophageal mucosa comprises radiofrequency energy. The radiofrequency energy can be delivered from the energy delivery device in a number of ways. Usually, the radiofrequency energy will be delivered in a bipolar fashion from a bipolar array of electrodes positioned on the energy-delivering device, typically on an expandable structure, such as a balloon, frame, cage, or the like, which can expand and deploy the electrodes directly against or immediately adjacent to the mucosal tissue (e.g., through direct contact or through a dielectric membrane or other layer). Alternatively, the energy delivery device may comprise a monopolar electrode structure which is energized by a radiofrequency power supply in combination with a return electrode typically positioned on the patient's skin, e.g., on the small of the back. In either case, the radiofrequency energy will typically be delivered at a high energy flux over a very short period of time in order to injure or ablate only the mucosal layer without substantially heating or otherwise damaging the submucosal tissue.

In particular examples the radiofrequency energy is delivered by an array of bipolar electrode pairs formed over the expandable structure, where each electrode typically has a width of less than 3 mm, usually in the range from 0.1 mm to 3 mm, preferably from 0.1 mm to 0.3 mm, and where the adjacent electrodes are spaced apart by a interelectrode distance of less than 3 mm, usually in the range from 0.1 mm to 3 mm, typically from 0.1 mm to 0.3 mm. Usually, the interelectrode spacing will be approximately equal to the widths of the adjacent electrodes. In the illustrated examples the widths of all electrodes will usually be equal, but it would be possible to use individual electrodes having different widths within the ranges set forth above. In all cases, the very close spacing of the bipolar electrode pairs assures that the current and resulting ohmic heating of the tissue is limited to a very shallow depth so that injury and heating of the submucosal layer are minimized.

In an example the expandable structure is a non-distensible balloon which provides a dimensionally stable carrier for the electrode array. Thus, as the balloon is expanded, the spacing and configuration of the electrodes on the non-distensible balloon remain constant.

Usually, the electrodes will be configured on the balloon or other expandable structure so that they directly contact the mucosal tissue when deployed. In other instances, however, the electrodes may be positioned on the inside surface of the balloon or other structure so that they do not directly engage the tissue. When not directly engaging the tissue, the balloon or other structure will be composed of a dielectric material, and the radiofrequency energy will be delivered across the dielectric to provide the ohmic heating in the tissue.

Other preferred methods rely on delivering thermal energy to the mucosal layer. Preferably, the thermal energy is transferred by radiation from a high temperature source, typically an element heated to a temperature above 1000°C. Suitable radiators include filaments, spherical radiators, cylindrical radiators, polygonal radiators, and the like. The radiators will be positioned to avoid direct contact with the esophageal wall. For example, the radiator may be positioned between two expansible balloons or other supports in order to align the radiator centrally within the esophageal lumen. Alternatively, the radiator may be positioned within a supporting balloon or other inflatable structure, where the structure is transparent to the radiated energy.

In a second aspect to the present disclosure, systems for treating mucosal tissue in an esophagus comprise an elongated member, an energy delivery structure, and an energy delivery source. The elongated member is typically in the form of a catheter or tube adapted to be delivered transorally into the esophagus. Often, the elongated member may include working channel(s), optical viewing capability, radiopaque markers, and other conventional structural features associated with esophageal probes.

The energy delivery structure may be adapted to deliver any of the energy sources listed above, including radiofrequency energy, thermal energy (including heating and cooling), microwave energy, ultraviolet energy, ultrasonic energy, light energy, and the like. The nature of the energy delivery apparatus will depend on the type of energy being delivered through the energy delivery structure. In the case of radiofrequency energy, the energy delivery apparatus will comprise a radiofrequency power supply together with control circuitry (analog or digital) which is adapted to deliver radiofrequency energy in the ranges and for the times described above. For thermal heating, the energy delivery apparatus will comprise a power supply, optionally including temperature control circuitry, for heating an associated heating element. Generally, for all energy delivery structures, it is expected that the total energy dosages per $cm^2$ delivered will be within the ranges set forth above.

The energy delivery structures of the treatment systems of the present disclosure will typically comprise an expandable structure, such as an expandable balloon, for engaging electrodes or other components of the energy delivery structure directly against the mucosal tissue, or in the case of high temperature or other radiative sources, spaced a distance from the mucosal wall. Usually, the structure will comprise a non-distensible and dimensionally stable balloon which carries the electrodes in a fixed geometry which does not change as the structure is expanded. Alternatively, the expansible structure may comprise an electrically conductive elastic balloon. In the latter case, the balloon will typically be filled with an electrolytic inflation material which permits it to be energized at a single polarity as part of a monopolar treatment system. The non-distensible, dimensionally stable expandable structures are used most often to position electrode arrays, either monopolar or bipolar, in a manner where their relative geometries do not vary.

A preferred electrode pattern on the non-distensible balloon or other dimensionally stable expandable structure comprises bipolar electrode pairs having a spacing therebetween which is no more than 3 mm. The bipolar electrode pairs may be aligned axially or circumferentially over the balloon or other expandable structure. Alternatively, the electrodes may be non-linear and arranged in a variety of other patterns so long as the interelectrode spacing remains within the 3 mm requirement.

As an alternative to the balloon, the expandable structure may comprise a frame which is deployable from the elongate member. Usually, the frame will expand to an arcuate frame having a shape and/or dimensions which engage only a portion of the full circumference of the esophageal wall (i.e. a partial circumferential section of the surface of the esophagus). The arcuate frame can have a shape and/or dimensions configured to engage esophageal circumferential portions in the range from about 20 to 359°, with preferred ranges of about 20 to 180°, about 20 to 90° or about 20 to 45°. In this way, the expandable frame may be fully expanded with a preselected electrode spacing to permit controlled energy delivery. Sections of the esophagus may be then treated sequentially for fixed time periods, where the total energy delivered to the mucosal wall is tightly controlled. Alternatively, the arcuate structure need not expandable, but can have a fixed shape configured to engage a selectable portion of the circumference of the esophageal wall. In such examples the arcuate structure can be deployed using the elongate member or via another advancement means, e.g., an endoscope.

The systems of the present disclosure may also comprise thermal energy delivery structures, such as filaments, spherical radiators, cylindrical radiators, polygonal radiators, and the like.

## BRIEF DESCRIPTION OF THE DRAWINGS

FIG. 1 is a schematic view of portions of an upper digestive tract in a human.

FIG. 1A is a schematic view of an energy delivery system of the present disclosure used for the treatment of abnormal tissue in the esophagus.

FIG. 2 is a schematic view of a device of the disclosure in an expanded mode, within an esophagus.

FIG. 3 is a schematic view of a device of the disclosure.

FIG. 4 is a detailed view of the device of FIG. 3.

FIG. 5 is a view of a device of the disclosure.

FIG. 6 shows the electrode patterns of the device of FIG. 3.

FIG. 7 shows electrode patterns that may be used with a device of the disclosure.

FIG. 8 shows an alternative expandable structure and an electrode deployment device constructed in accordance with the principles of the present disclosure.

FIGS. 9A and 9B illustrate use of the device of FIG. 8 for sequentially treating circumferential segments of an esophagus according to the methods of the present disclosure.

FIG. 10 illustrates yet another alternative electrode deployment apparatus according the principles of the present disclosure.

FIG. 11 illustrates an alternative balloon electrode deployment device according to the principles of the present disclosure.

FIG. 12 illustrates a balloon inflatable with an electrically conductive inflation medium which can be utilized as a monopolar radiofrequency delivery device.

FIG. 13 illustrates a balloon having a substantially continuous conductive layer formed over its surface and useable as a monopolar electrode deployment device.

FIG. 14 illustrates a balloon composed of a dielectric material having substantially continuous electrode structure formed over its interior surface.

FIG. 15 illustrates a balloon having a plurality of axially elongate external electrodes formed over its exterior surface.

FIG. 16 illustrates a first thermal treatment catheter constructed in accordance with the principles of the present disclosure and comprising a cylindrical radiation heat element.

FIG. 17 illustrates a thermal treatment catheter similar to that of FIG. 16, but including a spherical radiation heat element.

FIG. 18 illustrates a filament deployment catheter constructed in accordance with the principles of the present disclosure.

FIGS. 19A and 19B illustrate use of the catheter of FIG. 18 and thermally treating an esophagus in accordance with the principles of the present disclosure.

DETAILED DESCRIPTION OF THE INVENTION

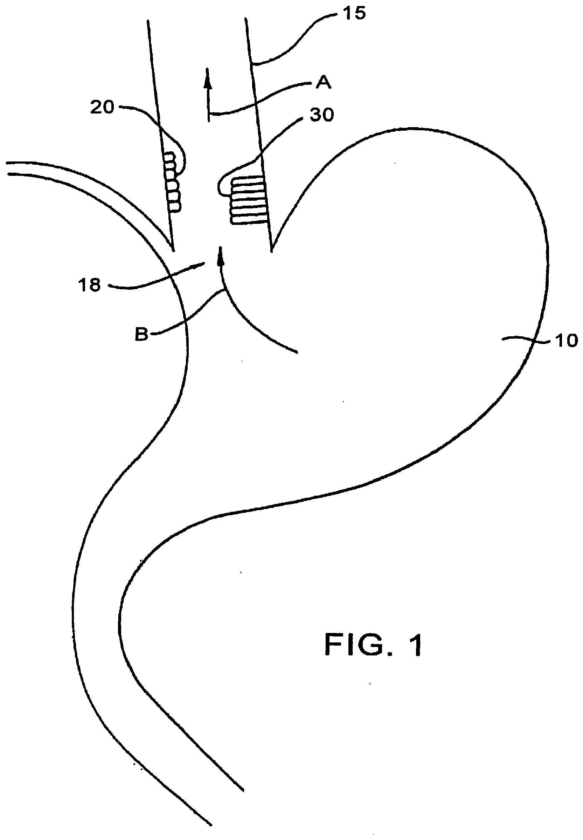

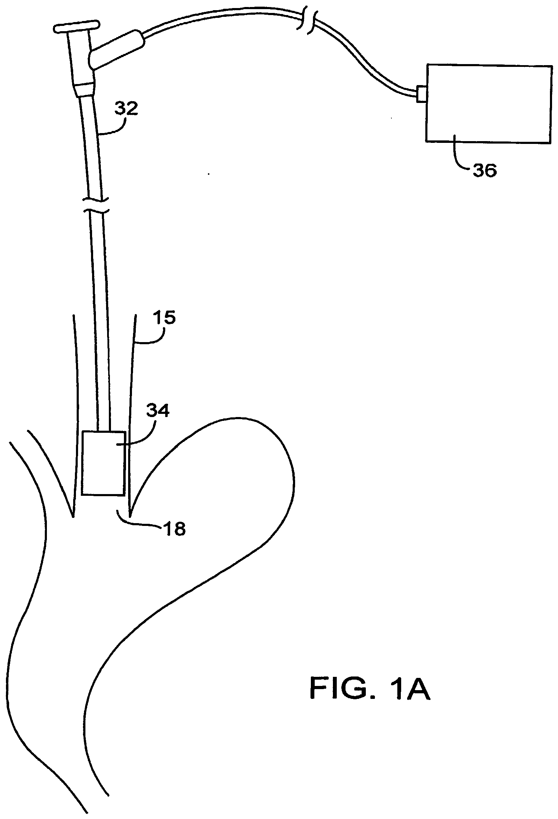

Various inflammatory disorders result in human patients who experience retrograde flow of gastric or intestinal contents from the stomach 10, as shown in FIG. 1, into the esophagus 15. This flow is shown by arrows A and B in FIG. 1. Although the causation of these problems are varied, this retrograde flow may result in secondary disorders which require treatment independent of and quite different from treatments appropriate for the primary disorder--such as disorders of the lower esophageal sphincter 18. One type of inflammatory disorder is known as Barrett's esophagus, in which the stomach acids, bile acids and enzymes regurgitated from the stomach and duodenum enter into the lower esophagus causing damage to the esophageal mucosa. Indeed, when this type of retrograde flow occurs frequently enough, damage may occur to esophageal epithelial cells 20. When normal replacement of damaged cells is overcome by the rate of damage, then the result may be symptomatic destruction of the healthy squamous epithelium. When this occurs, the squamous cells can be replaced by columnar epithelium 30 of the lower esophageal passageway. It is well established that although some of the columnar cells may be benign, others may result in adenocarcinoma. Accordingly, attention has been focused on identifying and removing this columnar epithelium in order to mitigate more severe implications for the patient. Examples of efforts to properly identify these growths, referred to as Barrett's epithelium or more generally as Barrett's esophagus, have included conventional visualization techniques known to practitioners in the field. Although certain techniques have been developed to characterize and distinguish such epithelium cells, such as disclosed in U.S. Pat. Nos. 5,524,622 and 5,888,743, there has yet to be shown efficacious means of accurately removing undesired growths of this nature from portions of the esophagus to mitigate risk of malignant transformation.

Systems for accomplishing this procedure according to this invention include the use of radiofrequency energy at conventional levels to accomplish injury or ablation of mucosal tissue, preferably without damage to submucosal tissue. Such treatment is designed to remove the columnar growths 30 from the portions of the esophagus 15 so effected. In particular, such treatment is designed to initiate the growth of new, normal mucosal cells to replace the columnar growths 30 and other abnormal cells in the mucosal layer of the esophagus. Referring now to FIG. 1 A, systems according to the present disclosure comprise an elongated member 32 having an energy delivery structure 34 at a distal end thereof. The energy delivery structure 34 is connected to an energy delivery supply 36, such as a radiofrequency or other power supply, which controls and delivers energy through the structure into the mucosal wall of the lower esophagus just above the lower esophageal sphincter 18. The energy delivery supply 36 is adapted to deliver energy at relatively high levels of power for relatively short periods of time in order to achieve the shallow depth of treatment desired. Preferred energy levels and treatment times have been set forth previously.

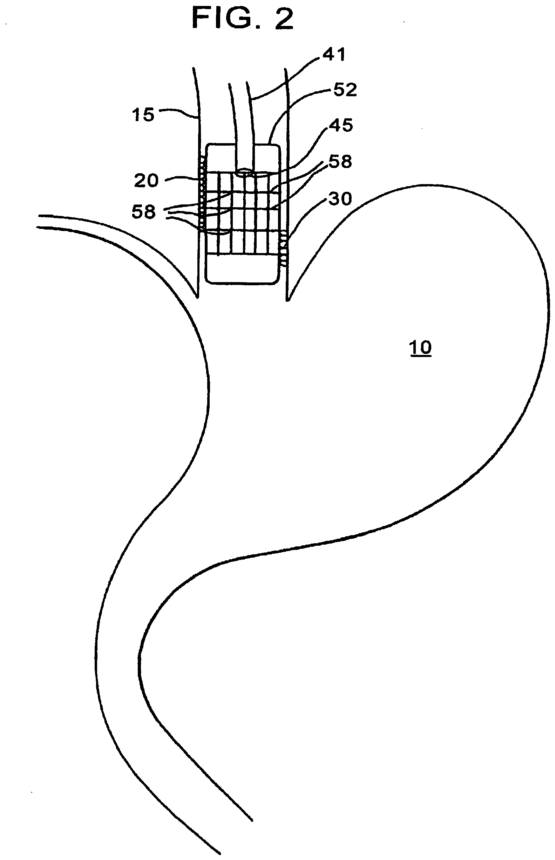

In a specific example as shown in FIG. 2, an elongated flexible shaft 41 is provided for insertion into the body in any of various ways selected by the medical provider. The shaft may be preferably placed endoscopically, e.g. through the esophagus, or it may be placed surgically, or by other means. Energy distribution means is provided at a distal end 45 of the flexible shaft to provide appropriate energy for ablation as desired. It is recognized that energy of a preferred type includes radiofrequency energy, microwave energy, or photonic or radiant sources such as infrared or ultraviolet light, the latter possibly in combination with improved sensitizing agents. Photonic sources can include semiconductor emitters, lasers, and other such sources. It is also recognized that another example of this disclosure may utilize heatable fluid as an ablation energy medium.

In one example the flexible shaft comprises a coaxial cable surrounded by an electrical insulation layer and comprises a radiant energy distribution means located at its distal end. In one form of the disclosure a positioning and distending device around the distal end of the instrument is of sufficient size to contact and expand the walls of the body cavity in which it is placed (e.g. the esophagus) both in the front of the distribution means as well as on the sides of the distribution means. For example, the distal head of the instrument can be supported at a controlled distance from the wall of the esophagus by an expandable balloon member 52 so as to regulate and control the amount of energy transferred to the tissue comprising the esophageal wall. The balloon is preferably bonded to a portion of the flexible shaft at a point spaced from the distal head means.

Another example comprises using the distending or expandable balloon member as the vehicle to deliver the ablation energy. A critical feature of this example includes means by which the energy is transferred from the distal head portion of the disclosure to the membrane comprising the balloon member. For example, one type of energy delivery means that may be appropriate and is shown in U.S. Pat. No. 5,713,942, in which an expandable balloon is connected to a power source which provides radiofrequency power having the desired characteristics to selectively heat the target tissue to a desired temperature. The balloon 52 of the current disclosure may be constructed of an electroconductive elastomer such as a mixture of polymer, elastomer, and electroconductive particles, or it may comprise a non-distensible bladder having a shape and a size in its fully expanded form which will extend in an appropriate way to the tissue to be contacted. In another example an electroconductive member may be formed from an electroconductive elastomer wherein an electroconductive material such as copper is deposited onto a surface and an electrode pattern is etched into the material and then the electroconductive member is attached to the outer surface of the balloon member. In one example the electroconductive member, e.g. the balloon member 52, has a configuration expandable in the shape to conform to the dimensions of the expanded (not collapsed) inner lumen of the human lower esophageal tract. In addition, such electroconductive member may consist of a plurality of electrode area segments 58 having thermistor means or the like associated with each electrode segment by which the temperature from each of a plurality of segments is monitored and controlled by feedback arrangement. In another example it is possible that the electroconductive member may have means for permitting transmission of microwave energy to the ablation site. In yet another example the distending or expandable balloon member may have means for carrying or transmitting a heatable fluid within one or more portions of the member so that the thermal energy of the heatable fluid maybe used as the ablation energy source.

A preferred device, such as that shown in FIG. 2, includes steerable and directional control means, a probe sensor for accurately sensing depth of cautery, and appropriate alternate examples so that in the event it is desired to not place the electroconductive elements within the membrane forming the expandable balloon member, it is still possible to utilize the balloon member for placement and location control while maintaining the energy discharge means at a location within the volume of the expanded balloon member, such as at a distal energy distribution head of conventional design.

In one example, the system disclosed herein may be utilized as a procedural method of treating Barrett's esophagus. This method includes the detection and diagnosis of undesired columnar epithelium within the esophagus. After determining that the portion or portions of the esophagus having this undesired tissue should be partially ablated, then the patient is prepared as appropriate according to the example of the device to be utilized. Then, the practitioner prepares the patient as appropriate and inserts, in one example, via endoscopic access and control, the ablation device shown and discussed herein through the mouth of the patient. Further positioning of portions of the device occur until proper location and visualization identifies the ablation site in the esophagus. Selection and activation of the appropriate quadrant(s) or portion(s)/segment(s) on the ablation catheter member is performed by the physician, including appropriate power settings according to the depth of treatment desired. Additional settings may be necessary as further ablation is required at different locations and/or at different depths within the patient's esophagus. Following the ablation, appropriate follow-up procedures as are known in the field are accomplished with the patient during and after removal of the device from the esophagus. The ablation treatment with ultraviolet light may also be accompanied by improved sensitizer agents, such as hematoporphyrin derivatives such as Photofrin® porfimer sodium, Johnson & Johnson Corporation, New Brunswick, N.J.).

In yet another aspect of the method the system disclosed herein may be utilized as a procedural method of treating dysplasia or cancerous tissue in the esophagus. After determining that the portion or portions of the esophagus having undesired tissue which should be partially ablated, then the patient is prepared as appropriate according to the embodiment of the device to be utilized and treatment is provided as described above.

In yet another method the practitioner may first determine the length of the portion of the esophagus requiring ablation and then may choose an ablation catheter from a plurality of ablation catheters of the invention, each catheter having a different length of the electrode member associated with the balloon member. For example, if the practitioner determined that 1 centimeter of the esophageal surface required ablation, an ablation catheter having 1 centimeter of the electrode member could be chosen for use in the ablation. The length of the electrode member associated with the balloon member can vary in length from 0.5 cm to 20 cm, usually from 1 cm to 10 cm.

In yet another example, a plurality of ablation catheters wherein the radiant energy distribution means are associated with the balloon member can be provided wherein the diameter of the balloon member when expanded varies from 12 mm to 50 mm. In this method, the practitioner will choose an ablation catheter having a diameter when expanded which will cause the esophagus to stretch and the mucosal layer to thin out, thus, reducing blood flow at the site of the ablation. The esophagus normally is 5 to 6 mm thick. In the method of the current disclosure, the esophagus is stretched and thinned so that the blood flow through the esophageal vasculature is occluded. It is believed that by reducing the blood flow in the area of ablation, the heat generated by the delivered energy is less easily dispersed to other areas of the esophagus, thus constraining the energy to the ablation site.

One means a practitioner may use to determine the appropriate diameter ablation catheter to use with a particular patient would be to use in a first step a highly compliant balloon connected to pressure sensing means. The balloon would be inserted into the esophagus and positioned at the desired site of the ablation and inflated until an appropriate pressure reading was obtained. The diameter of the inflated balloon would be determined and an ablation device of the disclosure having a balloon member capable of expanding to that diameter would be chosen for use in the treatment. In the method of this disclosure, it is desirable to expand the expandable electroconductive member such as a balloon sufficiently to occlude the vasculature of the submucosa, including the arterial, capillary or venular vessels. The pressure to be exerted to do so should therefore be greater than the pressure exerted by such vessels, typically from 7kPa to 138kPa relative to the surrounding atmosphere (1 psig to 20 psig), usually from 34kPa to 69kPa relative to the surrounding atmosphere (5 psig to 10 psig).

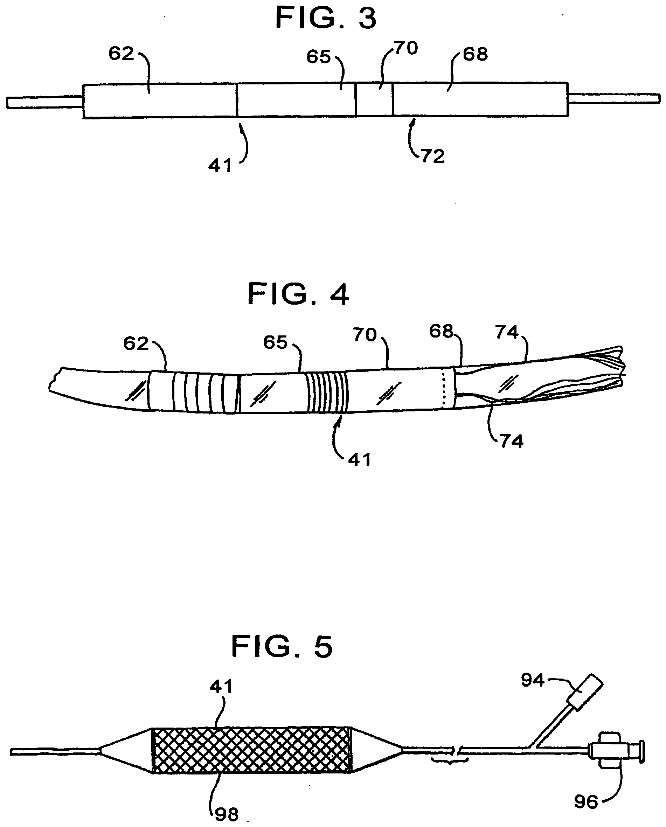

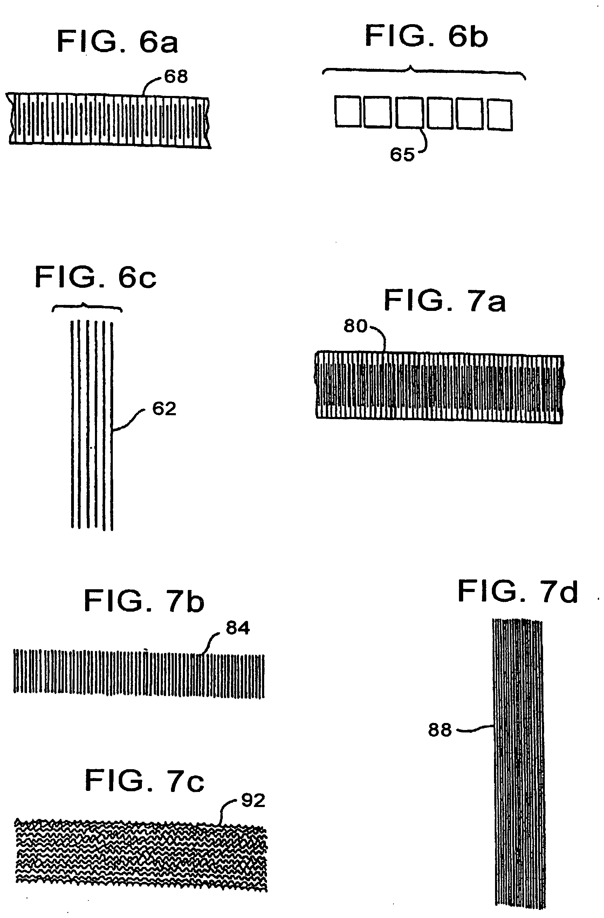

Operation and use of a device of disclosure are described as follows. The device used is shown schematically in FIGS. 3 and 5 and a detailed view of the device is shown in FIG. 4. As shown in FIG. 5, the elongated flexible shaft 41 is connected to a multi-pin electrical connector 94 which is connected to the power source and includes a male luer connector 96 for attachment to a fluid source useful in expanding the expandable member. The expandable member has an electrode 98 wrapped around the circumference. The expandable member of the device shown in FIGS. 3 and 4 further includes three different electrode patterns, the patterns of which are represented in greater detail in FIG. 6. Normally, only one electrode pattern would be used in a device of this disclosure. In this device, the elongated flexible shaft 41 comprises six bipolar rings 62 with 2 mm separation at one end of the shaft (one electrode pattern), adjacent to the bipolar rings is a section of six monopolar bands or rectangles 65 with 1 mm separation (a second electrode pattern), and another pattern of bipolar axial interlaced finger electrodes 68 is positioned at the other end of the shaft (a third electrode pattern). In this device, a null space 70 was positioned between the last of the monopolar bands and the bipolar axial electrodes. The catheter used in the study was prepared using a polyimide flat sheet of about 1 mil (0.001 ") thickness coated with copper. The desired electrode patterns were then etched into the copper.

The electrode patterns of the disclosure may vary, other possible electrode patterns are shown in FIG. 7 as 80, 84, 88, and 92, respectively. Pattern 80 is a pattern of bipolar axial interlaced finger electrodes with 0.3 mm separation. Pattern 84 includes monopolar bands with 0.3 mm separation. Pattern 88 includes bipolar rings with 0.3 mm separation. Pattern 92 is electrodes in a pattern of undulating electrodes with 0.2548 mm separation.

In this case the electrodes were attached to the outside surface of an esophageal dilation balloon 72 having a diameter of 18 mm. The device was adapted to use radiofrequency by attaching wires 74 as shown in FIG. 4 to the electrodes to connect them to the power source.

The balloon was deflated and the catheter inserted into the esophagus as described below. In addition to the series of three different electrode patterns a number of different energy factors were applied to the esophagus of a normal immature swine (about 25 kgs). First, an endoscope was passed into the esophagus of the subject. The device of the disclosure was placed into the distal esophagus using endoscopic guidance. The balloon member was inflated to press the electrodes against the esophageal mucosa. There was no indication that balloon dilation resulted in untoward effects on the esophagus.

Once the balloon member and electrodes were in place the first set of radiofrequency ("RF") applications were made. Following endoscopic evaluation of the treated areas, the device was withdrawn proximally. The placement of the device was evaluated endoscopically to assure a gap of normal tissue between the area of the first application and the second application, which gap will assure identification of the two treatment areas during post procedure evaluations. The procedure was repeated a third time using a similar procedure to that of the second application. During the treatment the tissue impedance was monitored as an indicator of the progress of the treatment, high impedance being an indication of desiccation. Accordingly, the practitioner can determine through monitoring the tissue impedance when sufficient ablation has occurred.

The RF energy can be applied to the electroconductive members in a variety of ways. In one example, it is applied in a bipolar mode to bipolar rings through simultaneous activation of alternating rings. In another example, it is applied to the bipolar rings through sequential activation of pairs of rings. In another example, the RF energy can be applied in monopolar mode through sequential activation of individual monopolar bands or simultaneous activation of the monopolar bands.

Various modifications to the above-mentioned treatment parameters can be made to optimize the ablation of the abnormal tissue. To obtain shallower lesions, the RF power applied may be increased while decreasing the treatment time. Also, the electrode patterns may be modified such as shown in FIG. 7 to improve the evenness and shallowness of the resulting lesions. The system and method of the disclosure may also be modified to incorporate temperature feedback, resistance feedback and/or multiplexing electrode channels.

As described above, the energy delivery structures have been adapted to deliver energy substantially simultaneously about the entire circumferential surface of a balloon or other expandable structure. When a balloon is inflated to different diameters, as will typically be required when treating different patients having different esophageal sizes, it will be appreciated that the electrode spacing on a surface of the balloon will vary. In order to achieve repeatable electrode spacings and resulting energy fluxes, therefore, it is been necessary to preselect balloon sizes which are capable of fully expanding to a known, fixed diameter of an esophagus of a particular patient. In this way, the electrode spacings are known, and energy delivery can be properly controlled. Such approaches are described in copending application no. 10/754,444 (published as US 7,150,745), the content of which may be of interest to the skilled reader

As an alternative to providing delivery structures having multiple sizes or otherwise maintaining a stable electrode spacing on an expansible balloon, the methods and apparatus of the present disclosure optionally provide for an electrode deployment structure which is intended to cover only a circumferential and/or axial segment of the esophagus. In that way, the structure can have a fixed electrode spacing or density which can then be advanced and repositioned over the esophageal surface in order to achieve complete coverage for a desired treatment.

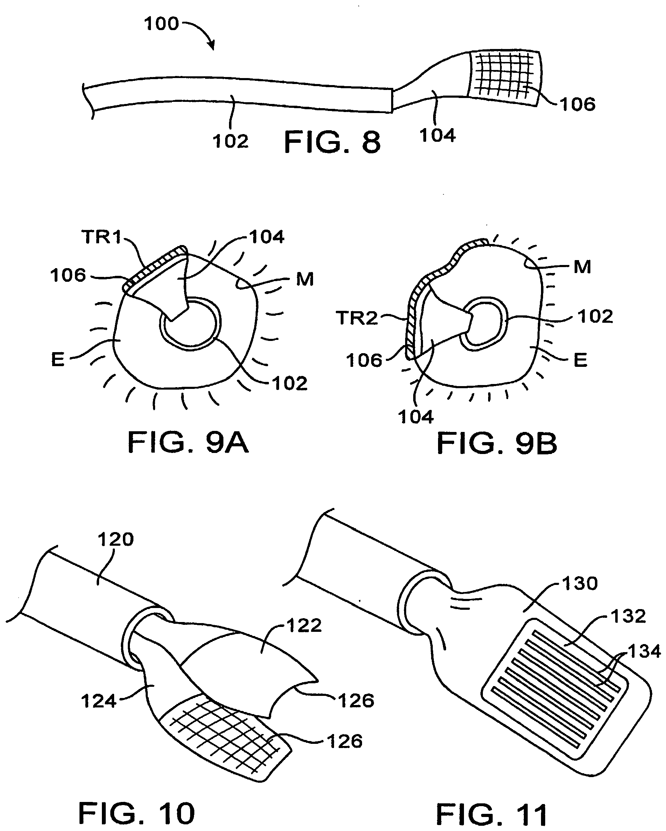

Referring now to FIG. 8, a catheter 100 comprising an elongated member 102, typically a tubular polymeric extrusion, includes a deployable arcuate electrode delivery structure 104. The arcuate frame can have a shape and/or dimensions configured to engage only a portion of the full circumference of the esophageal wall. In various examples, the engaged circumferential portion can be in the range from about 20 to 359°, with preferred ranges of about 20 to 180°, about 20 to 90° or about 20 to 45°. The structure may be collapsed by drawing it into the tube 102 or deployed by advancing it distally from the tube, as shown in FIG. 8. Alternatively, the arcuate structure need not expandable, but can have a fixed shape configured to engage a selectable portion of the circumference of the esophageal wall. An electrode array 106 is formed on an outer surface of the arcuate support 104 so that it can be engaged against a circumferential section of the mucosal lining M of the lower esophagus E, as shown in FIG. 9A. After radiofrequency energy is applied through the electrode array 106, a thin layer of mucosal tissue will be treated, as shown at TR1 in FIG. 9A. The arcuate deployment structure 104 may then be rotated so that the electrode array 106 is engaged against a new section of the mucosal wall of the esophagus E. A new region is treated, as shown at TR2 in FIG. 9B. By subsequently rotating the arcuate deployment structure 106, the entire circumferential wall of the esophagus may be treated. It would also be possible to axially reposition the catheter 100 in order to treat successive axial sections of the esophagus as well.

Referring now to FIG. 10, a treatment catheter 120, similar to catheter 100, may be provided with a pair of deployable arcuate structures 122 and 124. Electrode of arrays 126 may be formed on the outer and/or inner surfaces of the deployable arcuate structures. When formed over the inner surface, as shown in FIG. 10, the arcuate deployment structure will be composed of the dielectric material which permits radiofrequency treatment through the material into the esophageal wall. The use of a pair of deployment structures 122 and 124 reduces the total treatment time required when compared to the catheter 100 of FIG. 8.

A similar sequential treatment of the mucosal wall of an esophagus may be achieved using an inflatable balloon 130, as shown in FIG. 11. While most of the balloon 130 is formed from an elastomer, a non-distensible, dimensionally-stable portion 132 is formed as part of the balloon and carries a plurality of electrodes 134 spaced-apart in a fixed and dimensionally stable manner. Thus, the elastomeric balloon may be fully inflated in an esophagus and used to treat a selectable circumferential section of the esophagus. After an initial treatment is complete, the balloon may be rotated by a desired amount and used to treat the next circumferential section. Such sequential treatment is continued until the entire circumference of the esophagus has been treated. The dimensionally stable portion can have selectable shapes and radial dimensions to treat different sized portions of the esophagus. Accordingly, in various examples, the non-distensible portion can be configured to engage and treat esophageal circumferential portions in the range from about 20 to 359°, with preferred ranges of about 20 to 180°, about 20 to 90° or about 20 to 45°. Also, the dimensionally stable portion can be flatter than the elastic portion.

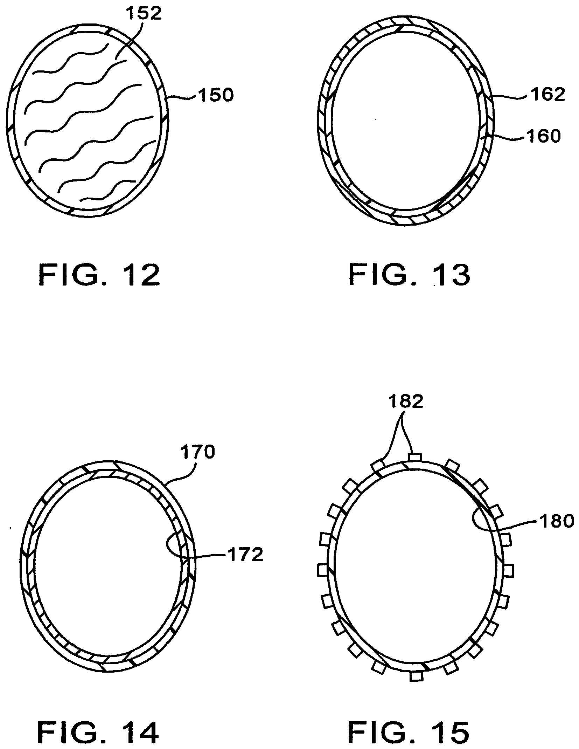

Referring now to FIGS. 12-15, a variety of monopolar radiofrequency electrode structures may be utilized in the systems of the present disclosure. Most simply, the treatment catheter may include a simple elastomeric or non-elastomeric balloon 150 formed from a dielectric or electrically conductive polymer. The balloon 150 may be filled with a conductive inflation medium, such as saline 152. The entire balloon may then be energized by one pole of a radiofrequency power supply in order to delivery energy through the balloon wall into the esophagus in which it has been inflated. A conventional return electrode is mounted on the patient's skin, typically on the lower back, in order to complete the "monopolar" treatment circuit. In FIG. 13, a simple elastomeric or non-distensible balloon 160 is covered with an electrically conductive coating 162 which can expand and deflate together with the balloon. The conductive layer 162 may be energized in a "monopolar" manner and utilized to treat a patient. In FIG. 14, a balloon 170, again either elastomeric or non-distensible, has an electrically conductive layer 172 formed on its interior surface. The conductive surface may be energized in a "monopolar" manner in the balloon used to treat patients. The balloon 170 is formed from dielectric or electrically conductive material, allowing treatment of the esophagus. In FIG. 15, balloon 180 includes a plurality of axially or otherwise oriented electrodes 182 formed over its exterior surface. Electrodes 182 may be energized in a "monopolar" manner by attachment to one pole with a suitable radiofrequency power supply.

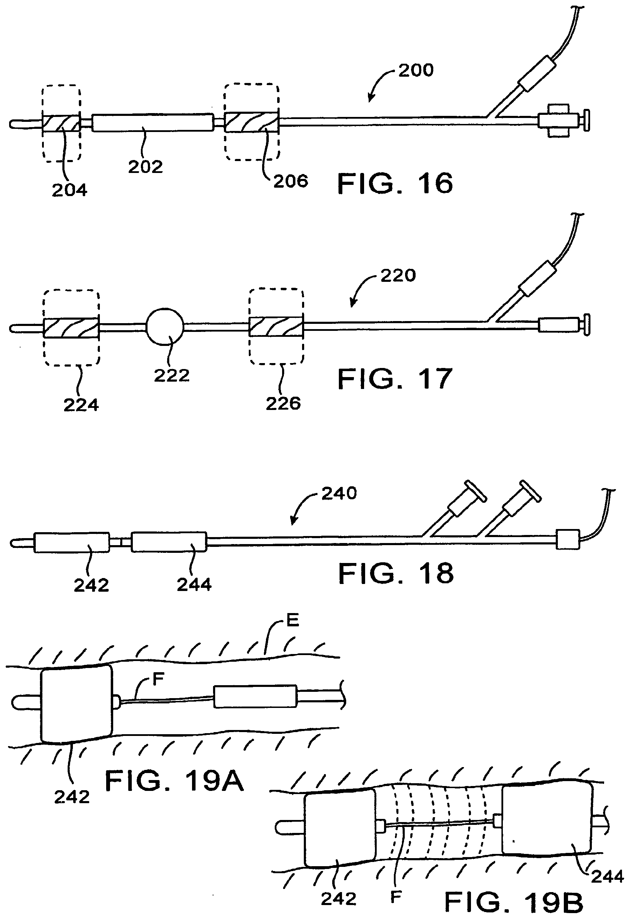

Turning now to FIGS. 16-19AB, use of a thermal delivery catheter in the methods and systems of the present disclosure will be described. A catheter 200, as shown in FIG. 16, comprises a cylindrical radiator structure 202, preferably disposed between a pair of expandable balloons 204 and 206 after the catheter has been located with its distal end within the lower esophagus. The balloons 204 and 206 may be inflated to center the energy delivery device 202 within the esophagus. The radiator 202 is then energized, typically by heating to a temperature of 1000°C or above, in order to radiate energy outwardly to heat, injure, and in some cases necrose, the tissue in the mucosal layer of the esophagus.

Catheter 220 of FIG. 17 is very similar to catheter 200 described above, except that a spherical radiator 222 is positioned between the first and second balloons 224 and 226, respectively.

Yet a further balloon catheter 240 is illustrated in FIG. 18, 19A, and 19B. The catheter 240 comprises expansible balloons 242 and 244. Initially, the balloons are spaced together as shown in FIG. 18A. After deployment of catheter 240 within an esophagus E, as shown in FIG. 19A, the first balloon 242 maybe inflated to anchor the distal end of the catheter. The body of catheter 240 is then pulled proximally, as shown in FIG. 19A, to expose a filament F for a preselected distance. As shown in FIG. 19B, once the filament F has been adequately exposed, the second balloon 244 will be inflated and used to anchor the filament firmly in place.

While a preferred embodiment of the present invention has been described, it should be understood that various changes, adaptations and modifications may be made therein without departing from the scope of the appended claims.

**Claims**

1. A system (100) for treating mucosal tissue in an esophagus, said system comprising:

   an elongated member (102);
   an energy delivery structure (104) comprising an arcuate surface configured to deploy by advancing from a distal end of the elongated member and collapse by retracting into the distal end of the elongated member;
   an electrode array (106) coupled to the arcuate surface of the energy delivery structure and configured to engage a surface of the esophagus; and
   means for delivering energy to the electrode array to ablate a mucosal layer of the engaged surface **characterized in that** the arcuate surface is a partially-circumferential arcuate surface.

2. The system as in claim 1, wherein the partially-circumferential arcuate surface spans less than about 180°.

3. The system as in claim 1, wherein the partially-circumferential arcuate surface spans less than about 90°.

4. The system as in claim 1, wherein the partially-circumferential arcuate surface spans between about 20° and about 180°.

5. The system as in claim 1, wherein the energy delivery structure is configured to be operable to sequentially treat different circumferential sections of the mucosal lining of the esophagus.

6. The system as in claim 1, wherein the energy delivery structure comprises an expandable structure comprising a frame deployable from the elongated member and the electrode array is formed over at least a portion of the frame.

7. The system as in claim 6, wherein the frame comprises two oppositely facing arcuate surfaces (126).

8. The system as in claim 1, wherein the means for delivering energy comprises a radiofrequency power supply (36).

9. The system as in claim 8, wherein the radiofrequency power supply is adaptable to deliver an energy dosage in the range form 1 J/cm$^2$ to 50 J/cm$^2$ over a time period less than 5 seconds.

10. The system as in claim 1, wherein the electrode array is a radiofrequency electrode array coupled to a radiofrequency power supply, the array positioned on a surface of the energy delivery structure.

11. The system as in claim 1, wherein the electrode array comprises an array of bipolar electrode pairs.

12. The system as in claim 11, wherein the electrodes have a width in the range from 0.1 mm to 3 mm and are spaced apart by a distance in the range from 0.1 mm to 3 mm.

**Patentansprüche**

1. System (100) zur Behandlung von Schleimhautgewebe in einer Speiseröhre, wobei das System Folgendes umfasst:

ein längliches Element (102);
eine Energieabgabestruktur (104), die eine gebogene Oberfläche umfasst, die dafür konfiguriert ist, sich durch das Vorschieben von einem distalen Ende des länglichen Elementes aufzustellen und durch das Zurückziehen in das distale Element des länglichen Elementes zusammenzufalten;
eine Elektrodenanordnung (106), die an die gebogene Oberfläche der Energieabgabestruktur gekoppelt ist und dafür konfiguriert ist, eine Oberfläche der Speiseröhre in Eingriff zu nehmen; und
Mittel zur Abgabe von Energie an die Elektrodenanordnung zum Abtragen einer Schleimhautschicht der in Eingriff genommenen Oberfläche,
**dadurch gekennzeichnet, dass** die gebogene Oberfläche eine teilweise umlaufende gebogene Oberfläche ist.

2. System nach Anspruch 1, wobei die teilweise umlaufende gebogene Oberfläche weniger als etwa 180°C umspannt.

3. System nach Anspruch 1, wobei die teilweise umlaufende gebogene Oberfläche weniger als etwa 90 °C umspannt.

4. System nach Anspruch 1, wobei die teilweise umlaufende gebogene Oberfläche zwischen etwa 20 °C bis etwa 180°C umspannt.

5. System nach Anspruch 1, wobei die Energieabgabestruktur derart konfiguriert ist, dass sie bedienbar ist, um sequentiell unterschiedliche Umfangsbereiche der Schleimhautauskleidung der Speiserohre zu behandeln.

6. System nach Anspruch 1, wobei die Energieabgabestruktur eine expandierbare Struktur umfasst, die einen Rahmen umfasst, der von dem länglichen Element einsetzbar ist, und die Elektrodenanordnung über mindestens einem Teil des Rahmens gebildet ist.

7. System nach Anspruch 6, wobei der Rahmen zwei sich gegenüberliegende gebogene Oberflächen (126) umfasst.

8. System nach Anspruch 1, wobei das Mittel zur Abgabe von Energie eine Radiofrequenz-Energieversorgung (36) umfasst.

9. System nach Anspruch 8, wobei die Radiofrequenz-Energieversorgung anpassbar ist, um eine Energiedosierung in der Form von 1 J/cm$^2$ bis 50 J/cm$^2$ über einen Zeitraum von weniger als 5 Sekunden abzugeben.

10. System nach Anspruch 1, wobei die Elektrodenanordnung eine Radiofrequenzelektrodenanordnung ist, mit einer Radiofrequenz-Energieversorgung gekoppelt ist, wobei die Anordnung auf einer Oberfläche der Energieabgabestruktur positioniert ist.

11. System nach Anspruch 1, wobei die Elektrodenanordnung eine Anordnung von bipolaren Elektrodenpaaren umfasst.

12. System nach Anspruch 11, wobei die Elektroden eine Breite im Bereich von 0,1 mm bis 3 mm aufweisen und durch einen Abstand im Bereich von 0,1 mm bis 3 mm beabstandet sind.

**Revendications**

1. Système (100) pour traiter un tissu muqueux dans un oesophage, ledit système comprenant :

un élément allongé (102) ;
une structure de fourniture d'énergie (104) comprenant une surface arquée configurée pour se déployer en avançant d'une extrémité distale de l'élément allongé et pour s'affaisser en se rétractant dans l'extrémité distale de l'élément allongé ;
un réseau d'électrodes (106) couplé à la surface arquée de la structure de fourniture d'énergie et configuré pour s'engager sur une surface de l'oesophage, et
des moyens pour délivrer de l'énergie au réseau d'électrodes afin d'effectuer l'ablation d'une couche muqueuse de la surface engagée, **caractérisé en ce que** la surface arquée est une surface arquée partiellement circonférentielle.

2. Système selon la revendication 1, dans lequel la surface arquée partiellement circonférentielle couvre moins d'environ 180°.

3. Système selon la revendication 1, dans lequel la surface arquée partiellement circonférentielle couvre moins d'environ 90°.

4. Système selon la revendication 1, dans lequel la surface arquée partiellement circonférentielle couvre entre environ 20° et environ 180°.

5. Système selon la revendication 1, dans lequel la structure de fourniture d'énergie est configurée pour être à même de traiter en séquence différentes sections circonférentielles de la muqueuse de l'oesophage.

6. Système selon la revendication 1, dans lequel la structure de fourniture d'énergie comprend une structure expansible comprenant un cadre déployable de l'élément allongé et le réseau d'électrodes est formé sur au moins une partie du cadre.

7. Système selon la revendication 6, dans lequel le cadre comprend deux surfaces arquées (126) en regard l'une de l'autre.

8. Système selon la revendication 1, dans lequel les moyens de fourniture d'énergie comprennent une alimentation en énergie à fréquence radio (36).

9. Système selon la revendication 8, dans lequel l'alimentation en énergie à fréquence radio est adaptable pour délivrer une dose d'énergie dans la plage de 1J/cm$^2$ à 50 J/cm$^2$ sur une période de temps inférieure à 5 secondes.

10. Système selon la revendication 1, dans lequel le réseau d'électrodes est un réseau d'électrodes à fréquence radio couplé à une alimentation en énergie à fréquence radio, le réseau étant positionné sur une surface de la structure de fourniture d'énergie.

11. Système selon la revendication 1, dans lequel le réseau d'électrodes comprend un réseau de paires d'électrodes bipolaires.

12. Système selon la revendication 11, dans lequel les électrodes ont une largeur dans la plage de 0,1 mm à 3 mm et sont séparées d'une distance dans la plage de 0,1 mm à 3 mm.

FIG. 1

FIG. 1A

# FIG. 2

# FIG. 3

# FIG. 4

# FIG. 5

# FIG. 6a

68

# FIG. 6b

65

# FIG. 6c

62

# FIG. 7a

80

# FIG. 7b

84

# FIG. 7d

88

# FIG. 7c

92

**FIG. 8**

**FIG. 9A**

**FIG. 9B**

**FIG. 10**

**FIG. 11**

FIG. 12

FIG. 13

FIG. 14

FIG. 15

FIG. 16

FIG. 17

FIG. 18

FIG. 19A

FIG. 19B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03092609 A2
- US 6405732 B1
- US 5524622 A
- US 5888743 A
- US 5713942 A
- US 10754444 B
- US 7150745 B